(19) Europäisches Patentamt
European Patent Office
Office européen des brevets

(11) Veröffentlichungsnummer: **0 213 403**
**B1**

(12) # EUROPÄISCHE PATENTSCHRIFT

(45) Veröffentlichungstag der Patentschrift: **11.07.90**

(51) Int. Cl.[5]: **C07C 211/21**

(21) Anmeldenummer: **86110552.6**

(22) Anmeldetag: **30.07.86**

(54) **Kontinuierliches Verfahren zur Herstellung von Dimethyldiallylammoniumchlorid.**

(30) Priorität: **13.08.85 DE 3528985**

(43) Veröffentlichungstag der Anmeldung: **11.03.87 Patentblatt 87/11**

(45) Bekanntmachung des Hinweises auf die Patenterteilung: **11.07.90 Patentblatt 90/28**

(84) Benannte Vertragsstaaten: **BE CH DE FR GB IT LI NL SE**

(56) Entgegenhaltungen: **DE-A- 2 739 131**

(73) Patentinhaber: **HOECHST AKTIENGESELLSCHAFT, Postfach 80 03 20, D-6230 Frankfurt am Main 80(DE)**

(72) Erfinder: **Aigner, Rudolf, Hochkalterstrasse 7, D-8269 Burgkirchen(DE)**
Erfinder: **Blaschke, Günter, Dr., Bajuwarenstrasse 35, D-8261 Winhöring(DE)**
Erfinder: **Müller, Günther, Dr., Kampenwandstrasse 13, D-8269 Burgkirchen(DE)**

**EP 0 213 403 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein kontinuierliches Verfahren zur Herstellung von Dimethyldiallylammoniumchlorid durch Umsetzung von Dimethylamin, Allylchlorid und in Wasser gelöstem Alkalimetallhydroxid unter Rühren.

Dimethyldiallylammoniumchlorid oder Diallyldimethylammoniumchlorid (im folgenden auch DADMAC genannt) ist eine wertvolle Verbindung, insbesondere im Hinblick auf die Herstellung von wasserlöslichen und leitfähigen Polyammoniumverbindungen. So stellen Homo- und Copolymerisate von DADMAC vorteilhafte Antistatika, Demulsifier, Flockungsmittel, Papierhilfsmittel und dergleichen dar. Die Homo- und Copolymerisate weisen aber nur dann die gewünschten Eigenschaften auf, wenn ein sehr reines DADMAC zur Polymerisation eingesetzt worden ist.

Es sind bereits eine Reihe von Verfahren zur Herstellung von Dimethyldiallylammoniumchlorid beschrieben worden. Bei den bekannten diskontinuierlichen Verfahren werden die Ausgangsverbindungen, das sind Dimethylamin, Allylchlorid und Alkali, im wesentlichen nacheinander und mehr oder weniger langsam zusammengebracht, wobei die drei Ausgangsverbindungen in einer bestimmten Reihenfolge eingesetzt, bestimmte Reaktionstemperaturen und bestimmte pH-Werte eingehalten und eine oder mehrere der Ausgangsverbindungen nicht in stöchiometrischer Menge, sondern in einem Überschuß eingesetzt werden, vgl. US-Patentschriften 2 923 701, 3 461 163 und 4 151 202.

Diese komplizierte Reaktionsführung wird deshalb für notwendig gehalten, weil andernfalls unerwünschte Nebenprodukte, wie insbesondere Allylalkohol (durch Verseifung oder Hydrolyse von Allylchlorid) gebildet werden. Es wird befürchtet, daß sich Allylalkohol vor allem dann bildet, wenn zu irgendeinem Zeitpunkt der Reaktion, insbesondere zu Beginn oder in der ersten Phase, eine vergleichsweise große Menge an Alkali zugegen ist.

Auch bei den bekannten kontinuierlichen Verfahren zur Herstellung von Diallyldimethylammoniumchlorid werden die Ausgangsverbindungen im wesentlichen nacheinander zusammengebracht, weil man auch hier beim gleichzeitigen Zusammenbringen von Alkali und Allylchlorid die genannte Verseifungsreaktion befürchtet. So werden bei dem aus der DD-Patentschrift 136 497 (Derwent-Publikation Nr. 70042B/39) bekannten kontinuierlichen Herstellungsverfahren von Diallyldimethylammoniumchlorid mit Hilfe eines Strömungsreaktors (Strömungsrohres) das Dimethylamin und die Alkalilauge nicht an der gleichen Stelle des Reaktors, in dem sich das Allylchlorid befindet, kontinuierlich zudosiert, sondern an zwei räumlich voneinander getrennten Stellen. Im Strömungsrohr wird eine Temperatur von 10 bis 30 °C und eine Verweilzeit von 5 bis 30 Minuten eingehalten. Wenn auch dieses Verfahren schon aufgrund seines kontinuierlichen Charakters gegenüber einem diskontinuierlichen gewisse Vorteile aufweist, haften ihm dennoch eine Reihe von Nachteilen an. Die Umsetzung im Strömungsreaktor läuft nicht bis zur vollständigen Quaternisierung ab, weshalb die gewünschte hohe Ausbeute nicht erreicht wird. Da bei der Umsetzung eine große Menge an Kochsalz entsteht, das in fester Form anfällt und zusammen mit dem gewünschten Endprodukt in Suspension vorliegt, ist es ein erhebliches Problem, bei dem beschriebenen Rohrreaktor Salzablagerungen zu vermeiden. Wegen der hohen Reaktionswärme, die aus der Umsetzung resultiert, ist die Wärmeabführung in der einzuhaltenden relativ kurzen Verweilzeit schwierig. Der für die Umsetzung benötigte Strömungsreaktor ist technisch kompliziert, weil er mit speziellen Prallkörpern, ansteigenden Strömungsrohren, die über Winkelstücke miteinander verbunden sind, und mit im Reaktor eingebauten Düsen ausgerüstet sein muß. Dieser spezielle Aufbau des Reaktors ist nicht zuletzt deswegen erforderlich, weil das Amin und die Alkalilauge an verschiedenen Stellen des Reaktors zudosiert werden sollen.

Der Erfindung liegt demnach die Aufgabe zugrunde, ein kontinuierliches Verfahren zur Herstellung von Diallyldimethylammoniumchlorid zur Verfügung zu stellen, das bei Einsatz der Ausgangsverbindungen in einer jeweils stöchiometrischen Menge sehr reines DADMAC (ohne daß dazu spezielle Reinigungsoperationen notwendig wären) in einer hohen Ausbeute liefert. Das Verfahren soll darüber hinaus in einer technisch einfachen Apparatur und unter Einsatz des Dimethylamins auch in Form von Flüssiggas durchgeführt werden können.

Das erfindungsgemäße Verfahren zur Herstellung von Dimethyldiallylammoniumchlorid durch Umsetzung von Dimethylamin, Allylchlorid und in Wasser gelöstem Alkalimetallhydroxid unter Rühren ist dadurch gekennzeichnet, daß die Umsetzung in mindestens zwei kaskadenförmig angeordneten Rührkesseln durchgeführt und dabei so vorgegangen wird, daß

a) Dimethylamin und Allylchlorid in jeweils stöchiometrischer Menge und nur 60 bis 95 Mol-% von der stöchiometrisch erforderlichen Menge an Alkalimetallhydroxid gleichzeitig und kontinuierlich dem ersten Rührkessel zugeführt werden und in diesem Kessel bei einer Temperatur von 20 bis 70 °C und dem sich ergebenden Druck eine solche Verweilzeit eingestellt wird, daß das den Kessel verlassende Produkt noch höchstens 10 Mol-% an freiem Amin, bezogen auf die eingesetzte Molmenge an Dimethylamin, enthält,

b) die beim ersten Rührkessel im Vergleich zur stöchiometrischen Menge verbleibende Restmenge an Alkalimetallhydroxid kontinuierlich den auf den ersten Kessel nachfolgenden Rührkesseln zugeführt wird und in jedem dieser Kessel bei einer im Bereich von 20 bis 70 °C liegenden und um bis zu 30 °C höheren Temperatur als im jeweils vorangehenden Kessel und dem sich ergebenden Druck eine solche Verweilzeit eingestellt wird, daß das den Kessel verlassende Produkt noch jeweils höchstens 2 Mol-% an freiem Amin, bezogen auf die eingesetzte Molmenge an Dimethylamin, enthält,wobei diese Menge an freiem Amin im Falle von mehreren nachfolgenden Rührkesseln von Kessel zu Kessel gleich oder kleiner gehalten wird, und

c) aus dem die Rührkesselkaskade verlassenden Produkt das angestrebte Dimethyldiallylammoniumchlorid gewonnen wird.

Nachdem im gesamten vorliegenden Stand der Technik zur Herstellung von reinem DADMAC abgeraten wird, die drei Ausgangsverbindungen gleichzeitig zusammenzubringen, war es überraschend, daß mit dem erfindungsgemäßen Verfahren, bei dem doch von dem stöchiometrisch erforderlichen Alkalimetallhydroxid nahezu die Gesamtmenge und vom Dimethylamin und Allylchlorid die ganze stöchiometrische Menge gleichzeitig zusammengebracht werden, ein sehr reines DADMAC erhalten wird. Es hat sich herausgestellt, daß die vom Stand der Technik befürchtete Verseifungsreaktion zwischen Allylchlorid und dem Alkali in der ersten Phase der Umsetzung der drei Ausgangsverbindungen nicht in nennenswertem Ausmaß eintritt; dies ist angesichts des bisher Offenbarten über die Umsetzung von Dimethylamin, Allylchlorid und Alkalilauge in der Tat ein unerwartetes Ergebnis. Im gesamten Stand der Technik zur Herstellung von DADMAC wird offensichtlich angenommen, daß im Reaktionsmechanismus, dem die Bildung von DADMAC zugrundeliegt, bestimmte unerwünschte Reaktionen bevorzugt und besonders schnell ablaufen. Dies ist, wie festgestellt worden ist, gerade in der ersten Phase der in Rede stehenden Umsetzung nicht der Fall. Es ist offensichtlich vielmehr so, daß bis zu einem relativ hohen Umsatz die Zwischenstufe Allyldimethylamin rasch übersprungen wird, das heißt, daß auf die erste Alkylierungsreaktion rasch die Quaternisierung (die zweite Alkylierung) folgt, und es deswegen nicht notwendig ist, die Umsetzung der drei Ausgangsverbindungen auf die genannte Zwischenstufe hinzusteuern durch Einhalten bestimmter pH-Werte, bestimmter Temperaturen und/oder durch gezieltes Zusammenbringen der Ausgangsverbindungen.

Die in Rede stehende Umsetzung kann durch die folgenden Reaktionsgleichungen charakterisiert werden (als Alkalimetallhydroxid wird NaOH eingesetzt):

$$(CH_3)_2NH + CH_2{=}CH{-}CH_2Cl \longrightarrow (CH_3)_2\overset{\oplus}{N}H{-}CH_2{-}CH{=}CH_2Cl^{\ominus}$$

$$(CH_3)_2NH + (CH_3)_2\overset{\oplus}{N}H{-}CH_2{-}CH{=}CH_2Cl^{\ominus} \rightleftarrows (CH_3)_2NH_2^{\oplus}Cl^{\ominus} + (CH_3)_2N{-}CH_2{-}CH{=}CH_2$$

$$(CH_3)_2\overset{\oplus}{N}H{-}CH_2{-}CH{=}CH_2Cl^{\ominus} + NaOH \longrightarrow (CH_3)_2N{-}CH_2{-}CH{=}CH_2 + NaCl + H_2O$$

$$(CH_3)_2NH_2^{\oplus}Cl^{\ominus} + NaOH \longrightarrow (CH_3)_2NH + NaCl + H_2O$$

$$(CH_3)_2N{-}CH_2{-}CH{=}CH_2 + CH_2{=}CH{-}CH_2Cl \longrightarrow (CH_3)_2\overset{\oplus}{N}(CH_2{-}CH{=}CH_2)_2Cl^{\ominus}$$

Wird die Umsetzung von Dimethylamin, Allylchlorid und Alkali (z. B. NaOH) als Summengleichung geschrieben, so lautet diese wie folgt:

$$(CH_3)_2NH + 2\ CH_2{=}CH{-}CH_2Cl + NaOH \longrightarrow (CH_3)_2\overset{\oplus}{N}(CH_2{-}CH{=}CH_2)_2Cl^{\ominus} + NaCl + H_2O$$

Aus den obigen Reaktionsgleichungen ist ersichtlich, daß das im Reaktionsgemisch vorhandene freie Amin die Summe aus Dimethylamin und Dimethylallylamin ist. Aus der Summengleichung ist ersichtlich,

daß die stöchiometrischen Mengen für die in Rede stehende Umsetzung 1 mol Dimethylamin, 2 mol Allylchlorid und 1 mol Alkalimetallhydroxid sind.

Dem ersten Rührkessel werden vorzugsweise nur 70 bis 90 % von der stöchiometrisch erforderlichen Molmenge an Alkalimetallhydroxid zugeführt.

Bezüglich der Reaktionstemperatur wird in den (auf den ersten Kessel) nachfolgenden Rührkesseln vorzugsweise die gleiche Temperatur wie im ersten Rührkessel oder eine um bis zu 30 °C höhere Temperatur als im ersten Rührkessel eingehalten; in diesem Falle ist bei mehreren nachfolgenden Kesseln die Temperatur von Kessel zu Kessel gleich oder in im wesentlichen gleichen Beträgen ansteigend. Es ist besonders bevorzugt, daß im ersten Rührkessel eine Temperatur von 30 bis 50 °C und in dem oder den nachfolgenden Rührkesseln eine Temperatur von 50 bis 60 °C eingehalten wird; bei mehreren nachfolgenden Rührkesseln ist die Temperatur von Kessel zu Kessel gleich oder in im wesentlichen gleichen Beträgen steigend.

Die beim ersten Rührkessel im Vergleich zur stöchiometrischen Menge verbleibende Restmenge an Alkalimetallhydroxid wird im Falle von nur einem nachfolgenden Rührkessel klarerweise diesem zugeführt. Im Falle von mehreren (auf den ersten Rührkessel) nachfolgenden Rührkesseln wird diese Restmenge vorzugsweise in von Kessel zu Kessel abnehmenden Portionen zugeführt. Dabei wird vorzugsweise so vorgegangen, daß den zwischen dem ersten und dem letzten Kessel stehenden Rührkesseln jeweils 50 bis 98 Mol-%, vorzugsweise jeweils 70 bis 95 Mol-%, von der aus dem vorangehenden Rührkessel verbleibenden Restmenge an Alkalimetallhydroxid zugeführt werden und dem letzten Rührkessel die ganze vom vorangehenden Kessel verbleibende Restmenge zugeführt wird.

Das den ersten Rührkessel verlassende Produkt enthält vorzugsweise noch 0,05 bis 10 Mol-%, insbesondere 0,1 bis 5 Mol-%, an freiem Amin, Molprozente bezogen auf die eingesetzte Molmenge an Dimethylamin. Das Produkt, das den oder die Rührkessel verläßt, die nach dem ersten Rührkessel angeordnet sind, enthält vorzugsweise noch 0,05 bis 2 Mol-%, insbesondere 0,1 bis 1 Mol-%, an freiem Amin, Molprozente bezogen auf die eingesetzte Molmenge an Dimethylamin. Im Falle von mehreren nachfolgenden Rührkesseln wird die Menge an freiem Amin jeweils gleich oder von Kessel zu Kessel kleiner gehalten; in diesem Falle nimmt die Menge an freiem Amin von Kessel zu Kessel vorzugsweise etwa gleichmäßig ab.

Das erfindungsgemäße Verfahren wird vorzugsweise in zwei oder drei kaskadenförmig angeordneten Rührkesseln durchgeführt.

Aus den oben angegebenen Maßnahmen geht hervor, daß bei einer bevorzugten Ausführungsform des erfindungsgemäßen Verfahrens die in Rede stehende Umsetzung in zwei oder drei kaskadenförmig angeordneten Rührkesseln durchgeführt und dabei so vorgegangen wird, daß

a) Dimethylamin und Allylchlorid in jeweils stöchiometrischer Menge und nur 70 bis 90 Mol-% von der stöchiometrisch erforderlichen Menge an Alkalimetallhydroxid gleichzeitig und kontinuierlich dem ersten Rührkessel zugeführt werden und in diesem Kessel bei einer Temperatur von 30 bis 50 °C und dem sich ergebenden Druck eine solche Verweilzeit eingestellt wird, daß das den Kessel verlassende Produkt noch 0,05 bis 10 Mol-%, vorzugsweise 0,1 bis 5 Mol-%, an freiem Amin enthält,

b) die beim ersten Rührkessel im Vergleich zur stöchiometrischen Menge verbleibende Restmenge an Alkalimetallhydroxid kontinuierlich dem nachfolgenden Rührkessel bzw. den beiden nachfolgenden Rührkesseln zugeführt wird und in jedem dieser Kessel bei einer Temperatur von 50 bis 60 °C und dem sich ergebenden Druck eine solche Verweilzeit eingestellt wird, daß das den Kessel verlassende Produkt noch jeweils 0,05 bis 2 Mol-%, vorzugsweise 0,1 bis 1 Mol-%, an freiem Amin enthält, wobei im Falle von 3 Rührkesseln im dritten die gleiche Temperatur wie im zweiten oder eine höhere Temperatur eingehalten wird und von der beim ersten Rührkessel verbleibenden Alkalimetallhydroxid-Restmenge dem zweiten Kessel 70 bis 95 Mol-% von dieser Restmenge und dem dritten Kessel die beim zweiten auf die stöchiometrische Alkalimetallhydroxid-Menge noch verbleibende Restmenge zugeführt wird, und

c) aus dem die Rührkesselkaskade verlassenden Produkt das angestrebte Dimethyldiallylammoniumchlorid gewonnen wird.

Beim erfindungsgemäßen Verfahren werden also die reagierenden Verbindungen, das sind Dimethylamin, Allylchlorid und Alkalimetallhydroxid, im stöchiometrisch erforderlichen Molverhältnis eingesetzt, nämlich im Molverhältnis 1 : 2 : 1 (vgl. die oben angeführten Reaktionsgleichungen).

Es versteht sich von selbst, daß möglichst reine Ausgangsverbindungen eingesetzt werden. Dimethylamin (Siedepunkt bei Normalbedingungen: 7 °C) kann als Flüssiggas, das heißt 100 %ig oder in Form einer wäßrigen Lösung eingesetzt werden, wobei 20 bis 50 gew.-%ige Lösungen bevorzugt sind. Allylchlorid (Siedepunkt bei Normalbedingungen: 44 °C)
wird als solches eingesetzt, da es in Wasser kaum löslich ist. Als Alkalimetallhydroxid wird vorzugsweise Natrium- und/oder Kaliumhydroxid eingesetzt. Die Konzentration der wäßrigen Lösung des Alkalimetallhydroxids (der Alkalilauge) beträgt 20 bis 80 Gew.-%, vorzugsweise 40 bis 60 Gew.-%. Die Menge (Gesamtmenge) des als Lösungsmittel eingesetzten Wassers wird zweckmäßigerweise so gewählt, daß die Konzentration an Wirkstoff, das heißt an DADMAC, in dem den letzten Rührkessel (die Kaskade) verlassenden Produkt (Reaktionsgemisch) 40 bis 75 Gew.-% beträgt, vorzugsweise 60 bis 70 Gew.-%.

Beim erfindungsgemäßen Verfahren beträgt die Verweilzeit (mittlere Verweilzeit)im Reaktionsraum, das heißt über alle Rührkessel in der Regel 10 bis 30 Stunden.

Bei den angegebenen Reaktionstemperaturen stellen sich Drücke von 0,2 bis 2 bar ein.

Die Bestimmung des freien Amins, das ist die Summe an Dimethylamin und Dimethylallylamin, kann beispielsweise durch Titration mit einer 0,1normalen (0,1n) Salzsäure unter Verwendung von Bromphenolblau als Indikator erfolgen (Bromphenolblau schlägt bei einem pH-Wert von 2,6 von blau auf gelb um).

Es ist zweckmäßig, in jedem Rührkessel auch die Menge an Hydrochlorid, das ist die Summe aus Dimethylaminhydrochlorid und Dimethylallylaminhydrochlorid zu bestimmen (sie resultiert aus der unterstöchiometrischen Alkalidosierung). Damit hat man beispielsweise eine Kontrolle bezüglich eventueller Ungenauigkeiten in der Menge der zudosierten Ausgangsverbindungen,wie Alkalimetallhydroxid. Diese Bestimmung erfolgt durch Titration mit einer 0,1normalen (0,1n) Natronlauge unter Verwendung von Thymolphthalein als Indikator (Thymolphthalein schlägt bei einem pH-Wert von 9,3 bis 10,5 von farblos nach blau um).

Die Aufarbeitung des beim erfindungsgemäßen Verfahren erhaltenen Produktes zur Gewinnung des angestrebten Diallyldimethylammoniumchlorids erfolgt nach an sich bekannten Methoden. Aus dem den letzten Rührkessel verlassenden Produkt wird zunächst Wasser abdestilliert (Aufkonzentrierung), zweckmäßigerweise im Wasserstrahlvakuum (Vakuum: etwa 10 bis 100 mbar, Temperatur: etwa 50 °C), wobei gleichzeitig evtl. vorhandene flüchtige Anteile wie Allylchlorid, Dimethylamin, Allylalkohol, Dimethylallylamin und dergleichen abgetrennt werden. Das Aufkonzentrieren, das zweckmäßigerweise unter Rühren durchgeführt wird, wird auch dann vorgenommen, wenn das aufzuarbeitende Produkt bereits die oben angegebenen DADMAC-Konzentrationen aufweist, um sicherzustellen, daß die genannten evtl. vorhandenen flüchtigen Anteile entfernt werden. Es ist zweckmäßig, dem die Kaskade verlassenden, aufzuarbeitenden Reaktionsgemisch einen im Vergleich zur stöchiometrischen Alkalimenge geringen Überschuß an Alkali, das ist ein Überschuß von etwa 0,5 Mol-%, zuzusetzen. Dieser Überschuß kann auch bereits im letzten Rührkessel zudosiert werden. Das nach dem Aufkonzentrieren erhaltene Produkt wird zentrifugiert (Temperatur: etwa 30 bis 50 °C), um das Alkalimetallchlorid abzutrennen. Das angestrebte Produkt (das Endprodukt) stellt eine vorzugsweise 60 bis 70 gew.-%ige wäßrige Lösung von DADMAC dar, die noch bis zu etwa 3 % Alkalimetallchlorid gelöst enthält (sofern bei der beschriebenen Aufarbeitung konzentriertere Lösungen an DADMAC anfallen, wird durch Zugabe von Wasser die angegebene gewünschte Konzentration eingestellt). Eine vollständige Befreiung von DADMAC von Wasser und Alkalimetallchlorid ist nicht erforderlich, weil bereits das genannte Endprodukt zu den eingangs genannten Zwecken eingesetzt werden kann.

Das erfindungsgemäße Verfahren weist eine Reihe von Vorteilen auf. Das angestrebte Dimethyldiallylammoniumchlorid wird in nahezu quantitativer Ausbeute erhalten. Die geringen Ausbeuteverluste resultieren im wesentlichen daher, daß beim erwähnten Zentrifugieren (oder Filtrieren) zur Abtrennung des größten Teils des anwesenden Alkalimetallhalogenids (das in der Regel Natriumchlorid ist) etwas DADMAC mitausgetragen wird. Die erhaltene wäßrige DADMAC-Lösung enthält keine die Polymerisation von DADMAC störende oder sogar hemmende Menge an Nebenprodukten mehr. Sie ist insbesondere praktisch frei von Allylalkohol und Allylchlorid. Dies wurde mit Hilfe der Headspace - Gaschromatographie festgestellt. Mit dem erfindungsgemäßen Verfahren ist es also möglich, Diallyldimethylammoniumchlorid in sehr hoher Ausbeute und in sehr hoher Reinheit herzustellen. Das erfindungsgemäße Verfahren ist kontinuierlich und kann in apparativ einfachen Rührkesseln durchgeführt werden. Daraus resultiert ein weiterer wesentlicher Vorteil, nämlich der,daß die in Rede stehende Umsetzung bezüglich betrieblicher Automatisierung, Steuerung, Auslastung und Einhaltung konstanter Umsetzungsbedingungen, was für eine gleichmäßige Produktqualität klarerweise von großer Bedeutung ist, optimal durchgeführt werden kann. Ein anderer wesentlicher Vorteil liegt darin, daß durch die in den Reaktionskesseln vorliegende hohe Konzentration an gebildetem Dimethyldiallylammoniumchlorid, das gute Lösungs- und Emulgiereigenschaften für Allylchlorid aufweist, eine nahezu homogene Reaktion erreicht wird, was beispielsweise zu höheren Reaktionsgeschwindigkeiten führt. Beim erfindungsgemäßen Verfahren sind auch keine aufwendigen Reinigungsoperationen, wie zum Beispiel die Reinigung mit Aktivkohle, erforderlich, da das angestrebte DADMAC in reinem Zustand erhalten wird.

Die Erfindung wird nun an Beispielen noch näher erläutert.

## Beispiel 1

Die Umsetzung wurde in zwei kaskadenförmig angeordneten Rührkesseln mit je 8 $m^3$ Volumen durchgeführt. Jeder der beiden Kessel war mit einem Rührer, einem Thermometer und einem Kühlmantel (Abführung der freiwerdenden Reaktionswärme mittels Kühlwasser) versehen.

In den ersten Rührkessel wurden über vier Einlaufstutzen pro Stunde kontinuierlich zugeführt: 265 kg (3,5 kmol) Allylchlorid, 78 kg (1,75 kmol) Dimethylamin und 84 kg 50 gew.-%ige wäßrige Natronlauge, das sind 1,05 kmol NaOH, und noch weiteres Wasser, nämlich 86 kg. Nachdem die stöchiometrische Menge an NaOH 1,75 kmol beträgt, wurden mit den 1,05 kmol NaOH in den ersten Rührkessel nur 60 Mol-% von der stöchiometrisch erforderlichen Menge zudosiert, das entspricht einem stöchiometrischen Alkaliunterschied von 0,7 kmol oder 40 Mol-%. Die beim ersten Rührkessel fehlende Alkalirestmenge von

0,7 kmol (das sind 56 kg 50 gew.-%ige Natronlauge) wurde pro Stunde kontinuierlich dem zweiten Rührkessel zugeführt.

Im ersten Rührkessel wurde eine Temperatur von 20 bis 25 °C und im zweiten Kessel eine Temperatur von 50 bis 55 °C gehalten (im ersten Kessel herrschte ein Druck von etwa 1,5 bar und im zweiten Kessel ein Druck von etwa 0,5 bar). Die mittlere Verweilzeit über beide Rührkessel betrug 28 Stunden.

Das den ersten Kessel verlassende Produkt (Produktgemisch) hatte einen Gehalt an freiem Amin von 9 Mol-% und das den zweiten Kessel verlassende Produkt einen Gehalt von 1,8 Mol-%, Molprozente bezogen auf das eingesetzte Dimethylamin. (Die Bestimmung des Gehaltes an freiem Amin, das ist die Summe aus Dimethylamin und Dimethylallylamin, erfolgte durch Titration mit einer 0,1 n Salzsäure mit Bromphenolblau als Indikator. In dem die beiden Rührkessel verlassenden Produktgemisch wurde auch der Gehalt an Hydrochlorid, das ist die Summe an Dimethylaminhydroohlorid und Dimethylallylaminhydrochlorid bestimmt; diese Bestimmung erfolgte durch Titration mit einer 0,1 nNatronlauge unter Verwendung von Thymolphthalein als Indikator und sie zeigte, daß ein Verlust oder ein Überschuß an Alkali nicht vorlag.)

Das den zweiten Rührkessel verlassende Produkt, das eine Wirkstoff-Konzentration, das ist die Konzentration an DADMAC, von 60 Gew.-% hatte, wurde zur weiteren Ausfällung von Natriumchlorid aufkonzentriert, wobei auch flüchtige Anteile wie freies Amin, Allylchlorid und dergleichen ausgetragen wurden. Dies erfolgte mit Hilfe eines Umlaufverdampfers, der bei einer Temperatur von 50 bis 60 °C und einem Druck von 80 bis 100 mbar betrieben wurde. Das nun vorliegende Produktgemisch (das eine Temperatur von etwa 55 °C hatte) wurde zentrifugiert, wobei das bei der Umsetzung entstandene und durch die Aufkonzentrierung zusätzlich auskristallisierte Natriumchlorid entfernt wurde.

Nach dem Zentrifugieren lag das angestrebte DADMAC vor, und zwar als wäßrige Lösung mit einem DADMAC-Gehalt von 70 Gew.-% und einem Natriumchlorid-Gehalt von 1,5 Gew.-% (Gewichtsprozente bezogen auf die wäßrige Lösung). Diese Lösung enthielt keine nennenswerten Mengen an Verunreinigungen wie Allylchlorid, Allylalkohol, freiem Amin und Aminhydrochlorid.

Beispiel 2

Die Umsetzung wurde in den zwei kaskadenförmig angeordneten Rührkesseln des Beispiels 1 durchgeführt.

In den ersten Rührkessel wurden pro Stunde kontinuierlich zugeführt: 353 kg (4,6 kmol) Allylchlorid, 104 kg (2,3 kmol) Dimethylamin und 129 kg 50 gew.-%ige wäßrige Natronlauge, das sind 1,6 kmol NaOH, und noch weiteres Wasser, nämlich 114 kg. Nachdem die stöchiometrische Menge an NaOH 2,3 kmol beträgt, wurden mit den 1,6 kmol NaOH in den ersten Rühr kessel nur 70 Mol-% von der stöchiometrisch erforderlichen Menge zudosiert, das entspricht einem stöchiometrischen Alkaliunterschied von 0,7 kmol oder 30 Mol-%. Die beim ersten Rührkessel verbleibende Alkali-Restmenge von 0,7 kmol (das sind 56 kg 50 %ige Natronlauge) wurde pro Stunde kontinuierlich dem zweiten Rührkessel zugeführt.

Im ersten Rührkesselwurde eine Temperatur von 40 bis 45 °C und im zweiten Kessel eine Temperatur von 50 bis 55 °C gehalten (im ersten Kessel herrschte ein Druck von etwa 1,5 bar und im zweiten Kessel ein Druck von etwa 0,5 bar).

Die mittlere Verweilzeit über beide Rührkessel betrug 21 Stunden.

Das den ersten Kessel verlassende Produkt (Produktgemisch) hatte einen Gehalt an freiem Amin von 4 Mol-% und das den zweiten Kessel verlassende Produkt einen Gehalt von 1 Mol-%, Molprozente bezogen auf das eingesetzte Dimethylamin.

Das den zweiten Rührkessel verlassende Produkt hatte eine Wirkstoff-Konzentration von 60 Gew.-% und wurde wie in Beispiel 1 aufgearbeitet.

Es lag eine von Verunreinigungen freie wäßrige Lösung mit einem DADMAC-Gehalt von 70 Gew.-% und mit einem Natriumchlorid-Gehalt von 1,5 Gew.-% vor.

Beispiel 3

Die Umsetzung wurde in den zwei kaskadenförmig angeordneten Reaktionskesseln des Beispiels 1 durchgeführt.

In den ersten Rührkessel wurden pro Stunde kontinuierlich zugeführt: 265 kg (3,5 kmol) Allylchlorid, 78 kg (1,75 kmol) Dimethylamin und 111 kg 50 gew.-%ige wäßrige Natronlauge, das sind 1,4 kmol NaOH, und noch weiteres Wasser, nämlich 86 kg. Nachdem die stöchiometrische Menge an NaOH 1,75 kmol beträgt,wurden mit den 1,4 kmol NaOH in den ersten Rührkessel nur 80 Mol-% von der stöchiometrisch erforderlichen Menge zudosiert, das entspricht einem stöchiometrischen Alkaliunterschuß von 0,35 kmol oder 20 Mol-%. Die beim ersten Rührkessel verbleibende Alkali-Restmenge von 0,35 kmol (das sind 29 kg 50 %ige Natronlauge) wurde pro Stunde kontiniuierlich dem zweiten Rührkessel zugeführt.

Im ersten Rührkessel wurde eine Temperatur von etwa 50 °C und im zweiten Kessel eine Temperatur von ebenfalls etwa 50 °C gehalten (im ersten Kessel herrschte ein Druck von etwa 1,5 bar und im zweiten Kessel ein Druck von etwa 0,5 bar).

Die mittlere Verweilzeit über beide Rührkessel betrug 28 Stunden.

Das den ersten Kessel verlassende Produkt (Produktgemisch) hatte einen Gehalt an freiem Amin von 0,3 Mol-% und das den zweiten Kessel verlassende Produkt einen Gehalt von 0,1 Mol-%, Molprozente bezogen auf das eingesetzte Dimethylamin.

Das den zweiten Rührkessel verlassende Produkt hatte eine Wirkstoff-Konzentration von 60 Gew.-% und wurde wie in Beispiel 1 aufgearbeitet.

Es lag eine von Verunreinigungen freie wäßrige Lösung mit einem DADMAC-Gehalt von 70 Gew.-% und mit einem Natriumchlorid-Gehalt von 1,5 Gew.-% vor.

Beispiel 4

Die Umsetzung wurde in drei kaskadenförmig angeordneten Reaktionskesseln mit je 8 $m^3$ Volumen durchgeführt. Jeder der drei Kessel war mit einem Rührer, einem Thermometer und einem Kühlmantel (Abführung der freiwerdenden Reaktionswärme mittels Kühlwasser) versehen.

In den ersten Rührkessel wurden über vier Einlaufstutzen pro Stunde kontinuierlich zugeführt: 662 kg (8,6 kmol) Allylchlorid, 195 kg (4,3 kmol) Dimethylamin und 258 kg 40 gew.-%ige wäßrige Natronlauge, das sind 2,6 kmol NaOH, und noch weiteres Wasser, nämlich 128 kg. Nachdem die stöchiometrische Menge an NaOH 4,3 kmol beträgt, wurden mit den 2,6 kmol NaOH in den ersten Rührkessel nur 60 Mol-% von der stöchiometrisch erforderlichen Menge zudosiert, das entspricht einem stöchiometrischen Alkaliunterschuß von 1,7 kmol oder 40 Mol-%. Von der beim ersten Rührkessel verbleibenden 1,7 kmol Alkali-Restmenge wurden 70 %, das sind 1,2 kmol NaOH, pro Stunde kontinuierlich dem zweiten Rührkessel und die beim zweiten Rührkessel auf die stöchiometrische Menge NaOH noch übrige Restmenge pro Stunde kontinuierlich dem dritten Reaktionskessel zugeführt.

Im ersten Rührkessel wurde eine Temperatur von 20 bis 25°C, im zweiten Kessel eine Temperatur von 40 bis 45 °C und im dritten Kessel eine Temperatur von 55 bis 60 °C gehalten (im ersten Kessel herrschte ein Druck von etwa 2,0 bar,im zweiten Kessel von etwa 1,5 bar und im dritten Kessel von etwa 0,5 bar).

Die mittlere Verweilzeit über die drei Rührkessel betrug 19 Stunden.

Das den ersten Kessel verlassende Produkt (Produktgemisch) hatte einen Gehalt an freiem Amin von 9 Mol-%, das den zweiten Kessel verlassende Produkt einen solchen von 2 Mol-% und das den dritten Kessel verlassende Produkt einen solchen von 1 Mol-%, Molprozente bezogen auf das eingesetzte Dimethylamin. (Die Bestimmung des Gehaltes an freiem Amin, das ist die Summe aus Dimethylamin und Dimethylallylamin, erfolgte wie in den Beispielen 1 bis 3. In dem die drei Rührkessel verlassenden Produktgemisch wurde auch der Gehalt an Hydrochlorid, das ist die Summe aus Dimethylaminhydrochlorid und Dimethylallylaminhydrochlorid, bestimmt; diese Bestimmung erfolgte ebenso wie in den Beispielen 1 bis 3 und sie zeigte, daß ein Verlust oder ein Überschuß an Alkali nicht vorlag.)

Das den dritten Reaktionskessel verlassende Produkt hatte eine Wirkstoff-Konzentration von 60 Gew.-% und wurde wie in den Beispielen 1 bis 3 aufgearbeitet.

Es lag eine von Verunreinigungen freie wäßrige Lösung mit einem DADMAC-Gehalt von 70 Gew.-% und mit einem Natriumchlorid-Gehalt von 1,5 Gew.-% vor.

Beispiel 5

Die Umsetzung wurde in den drei kaskadenförmig angeordneten Reaktionskesseln des Beispiels 4 durchgeführt.

In den ersten Rührkessel wurden pro Stunde kontinuierlich zugeführt: 530 kg (6,9 kmol) Allylchlorid, 156 kg (3,45 kmol) Dimethylamin und 259 kg 40 gew.-%ige wäßrige Natronlauge, das sind 2,6 kmol NaOH, und noch weiteres Wasser, nämlich 445 kg. Nachdem die stöchiometrische Menge an NaOH 3,45 kmol beträgt, wurden mit den 2,6 kmol NaOH in den ersten Rührkessel nur 75 Mol-% von der stöchiometrisch erforderlichen Menge zudosiert, das entspricht einem stöchiometrischen Alkaliunterschuß von 0,85 kmol oder 25 Mol-%. Von der beim ersten Rührkessel fehlenden 0,85 kmol Alkali-Restmenge wurden 80 %, das sind 0,7 kmol NaOH, pro Stunde kontinuierlich dem zweiten Rührkessel und die beim zweiten Rührkessel auf die stöchiometrische Menge NaOH noch übrige Restmenge pro Stunde kontinuierlich dem dritten Rührkessel zugeführt.

Im ersten Rührkessel wurde eine Temperatur von 30 bis 35 °C, im zweiten Kessel eine Temperatur von 40 bis 45 °C und im dritten Kessel eine Temperatur von 55 bis 60 °C gehalten (im ersten Kessel herrschte ein Druck von etwa 2,0 bar, im zweiten Kessel von etwa 1,5 bar und im dritten Kessel von etwa 0,5 bar).

Die mittlere Verweilzeit über die drei Rührkessel betrug 16 Stunden.

Das den ersten Kessel verlassende Produkt (Produktgemisch) hatte einen Gehalt an freiem Amin von 5 Mol-%, das den zweiten Kessel verlassende Produkt einen solchen von 1 Mol-% und das den dritten Kessel verlassende Produkt einen solchen von 0,5 Mol-%, Molprozente bezogen auf das eingesetzte Dimethylamin.

Das den dritten Reaktionskessel verlassende Produkt hatte eine Wirkstoff-Konzentration von 65 Gew.-% und wurde wie in den Beispielen 1 bis 3 aufgearbeitet.

Es lag eine von Verunreinigungen freie wäßrige Lösung mit einem DADMAC-Gehalt von 70 Gew.-% und mit einem Natriumchlorid-Gehalt von 1,5 Gew.-% vor.

Beispiel 6

Die Umsetzung wurde in den drei kaskadenförmig angeordneten Reaktionskesseln des Beispiels 4 durchgeführt.

In den ersten Rührkessel wurden pro Stunde kontinuierlich zugeführt: 397 kg (5,2 kmol) Allylchlorid, 117 kg (2,6 kmol) Dimethylamin und 177 kg 50 gew.-%ige wäßrige Natriumlauge, das sind 2,2 kmol NaOH, und noch weiteres Wasser, nämlich 268 kg. Nachdem die stöchiometrische Menge an NaOH 2,6 kmol beträgt, wurden mit dem 2,2 kmol NaOH in den ersten Rührkessel nur 85 Mol-% von der stöchiometrisch erforderlichen Menge zudosiert, das entspricht einem stöchiometrischen Alkaliunterschuß von 0,4 kmol oder 15 Mol-%. Von der beim ersten Rührkessel verbleibenden 0,4 kmol Alkali-Restmenge wurden 80 %, das sind 0,32 kmol NaOH pro Stunde kontinuierlich dem zweiten Rührkessel und die beim zweiten Rührkessel auf die stöchiometrische Menge NaOH noch übrige Restmenge pro Stunde kontinuierlich dem dritten Reaktionskessel zugeführt.

Im ersten Rührkessel wurde eine Temperatur von 40 bis 45 °C, im zweiten Rührkessel eine Temperatur von 50 bis 55 °C und im dritten Kessel eine Temperatur von 50 bis 55 °C gehalten (im ersten Kessel herrschte ein Druck von etwa 1,5 bar, im zweiten Kessel von etwa 1,0 bar und im dritten Kessel von etwa 0,5 bar). Die mittlere Verweilzeit über die drei Rührkessel betrug 24 Stunden.

Das den ersten Kessel verlassende Produkt (Produktgemisch) hatte einen Gehalt an freiem Amin von 0,5 Mol-%, das den zweiten Kessel verlassende Produkt einen solchen von 0,1 Mol-% und das den dritten Kessel verlassende Produkt einen solchen von 0,05 Mol-%, Molprozente bezogen auf das eingesetzte Dimethylamin. Das den dritten Reaktionskessel verlassende Produkt hatte eine Wirkstoff-Konzentration von 68 Gew.-% und wurde wie in den Beispielen 1 bis 3 aufgearbeitet.

Es lag eine von Verunreinigungen freie wäßrige Lösung mit einem DADMAC-Gehalt von 70 Gew.-% und mit einem Natriumchlorid-Gehalt von 1,5 Gew.-% vor.

Beispiel 7

Die Umsetzung wurde in den drei kaskadenförmig angeordneten Reaktionskesseln des Beispiels 4 durchgeführt.

In den ersten Rührkessel wurden pro Stunde kontinuierlich zugeführt: 397 kg (5,2 kmol) Allylchlorid, 117 kg (2,6 kmol) Dimethylamin und 234 kg 40 gew.-%ige wäßrige Natronlauge, das sind 2,34 kmol NaOH, und noch weiteres Wasser, nämlich 293 kg. Nachdem die stöchiometrische Menge an NaOH 2,6 kmol beträgt, wurden mit den 2,34 kmol NaOH in den ersten Rührkessel nur 90 Mol-% von der stöchiometrisch erforderlichen Menge zudosiert, das entspricht einem stöchiometrischen Alkaliunterschuß von 0,26 kmol oder 10 Mol-%. Von der beim ersten Rührkessel fehlenden 0,26 kmol Alkali-Restmenge wurden 95 %, das sind 0,25 kmol NaOH, pro Stunde kontinuierlich dem zweiten Rührkessel und die beim zweiten Rührkessel auf die stöchiometrische Menge NaOH noch übrige Restmenge pro Stunde kontinuierlich dem dritten Reaktionskessel zugeführt.

Im ersten Rührkessel wurde eine Temperatur von 55 bis 60 °C im zweiten Kessel eine Temperatur von 55 bis 60 °C und im dritten Kessel eine Temperatur von ebenfalls 55 bis 60 °C gehalten (im ersten Kessel herrschte ein Druck von etwa 1,5 bar, im zweiten Kessel von etwa 1,0 bar und im dritten Kessel von etwa 0,5 bar).

Die mittlere Verweilzeit über die drei Rührkessel betrug 22 Stunden.

Das den ersten Kessel verlassende Produkt (Produktgemisch) hatte einen Gehalt an freiem Amin von 0,5 Mol-%, das den zweiten Kessel verlassende Produkt einen solchen von 0,2 Mol-% und das den dritten Kessel verlassende Produkt einen solchen von 0,1 Mol-%, Molprozente bezogen auf das eingesetzte Dimethylamin.

Das den dritten Reaktionskessel verlassende Produkt hatte eine Wirkstoff-Konzentration von 68 Gew.-% und wurde wie in den Beispielen 1 bis 3 aufgearbeitet.

Es lag eine von Verunreinigungen freie wäßrige Lösung mit einem DADMAC-Gehalt von 70 Gew.-% und mit einem Natriumchlorid-Gehalt von 1,5 Gew.-% vor.

**Patentansprüche**

1. Kontinuierliches Verfahren zur Herstellung von Dimethyldiallylammoniumchlorid durch Umsetzung von Dimethylamin, Allylchlorid und in Wasser gelöstem Alkalimetallhydroxid unter Rühren, dadurch gekennzeichnet, daß die Umsetzung in mindestens zwei kaskadenförmig angeordneten Rührkesseln durchgeführt und dabei so vorgegangen wird, daß

a) Dimethylamin und Allylchlorid in jeweils stöchiometrischer Menge und nur 60 bis 95 Mol-% von der stöchiometrisch erforderlichen Menge an Alkalimetallhydroxid gleichzeitig und kontinuierlich dem ersten Rührkessel zugeführt werden und in diesem Kessel bei einer Temperatur von 20 bis 70 °C und dem sich ergebenden Druck eine solche Verweilzeit eingestellt wird, daß das den Kessel verlassende Produkt noch höchstens 10 Mol-% an freiem Amin, bezogen auf die eingesetzte Molmenge an Dimethylamin, enthält,

b) die beim ersten Rührkessel im Vergleich zur stöchiometrischen Menge verbleibende Restmenge an Alkalimetallhydroxid kontinuierlich den auf den ersten Kessel nachfolgenden Rührkesseln zugeführt wird und in jedem dieser Kessel bei einer im Bereich von 20 bis 70 °C liegenden und um bis zu 30 °C höheren Temperatur als im jeweils vorangehenden Kessel und dem sich ergebenden Druck eine solche Verweilzeit eingestellt wird, daß das den Kessel verlassende Produkt noch jeweils höchstens 2 Mol-% an freiem Amin, bezogen auf die eingesetzte Molmenge an Dimethylamin, enthält, wobei diese Menge an freiem Amin im Falle von mehreren nachfolgenden Rührkesseln von Kessel zu Kessel gleich oder kleiner gehalten wird, und

c) aus dem die Ruhrkessel-Kaskade verlassenden Produkt das angestrebte Dimethyldiallylammoniumchlorid gewonnen wird.

2. Verfahren nach Anspruch 1, dadurch gekennzeichnet, daß in den nachfolgenden Rührkesseln die gleiche Temperatur wie im ersten Rührkessel oder eine um bis zu 30 °C höhere Temperatur als im ersten Rührkessel eingehalten wird, wobei im Falle von mehreren nachfolgenden Rührkesseln die Temperatur von Kessel zu Kessel gleich ist oder in im wesentlichen gleichen Beträgen ansteigt.

3. Verfahren nach einem der Ansprüche 1 oder 2, dadurch gekennzeichnet, daß im ersten Rührkessel eine Temperatur von 30 bis 50 °C eingehalten wird und in den auf den ersten Rührkessel nachfolgenden Rührkesseln eine Temperatur von 50 bis 60 °C eingehalten wird, wobei im Falle von mehreren nachfolgenden Rührkesseln die Temperatur von Kessel zu Kessel gleich ist oder in im wesentlichen gleichen Beträgen ansteigt.

4. Verfahren nach einem oder mehreren der Ansprüche 1 bis 3, dadurch gekennzeichnet, daß die beim ersten Rührkessel im Vergleich zur stöchiometrischen Menge verbleibende Restmenge an Alkalimetallhydroxid den auf den ersten Kessel nachfolgenden Rührkesseln in von Kessel zu Kessel abnehmenden Portionen zugeführt wird.

5. Verfahren nach einem oder mehreren der Ansprüche 1 bis 4, dadurch gekennzeichnet, daß die Umsetzung in zwei oder drei kaskadenförmig angeordneten Rührkesseln durchgeführt wird.

6. Verfahren nach einem oder mehreren der Ansprüche 1 bis 5, dadurch gekennzeichnet, daß die Umsetzung in zwei oder drei kaskadenförmig angeordneten Rührkesseln durchgeführt wird und dabei so vorgegangen wird, daß dem ersten Rührkessel nur 70 bis 90 Mol-% von der stöchiometrisch erforderlichen Menge an Alkalimetallhydroxid zugeführt werden und in diesem Kessel bei einer Temperatur von 30 bis 50 °C und dem sich ergebenden Druck eine solche Verweilzeit eingestellt wird, daß das den Kessel verlassende Produkt noch 0,05 bis 10 Mol-% an freiem Amin enthält, und in jedem der auf den ersten Rührkessel nachfolgenden Rührkesseln bei einer Temperatur von 50 bis 60 °C und dem sich ergebenden Druck eine solche Verweilzeit eingestellt wird, daß das den Kessel verlassende Produkt noch jeweils 0,05 bis 2 Mol-% an freiem Amin enthält, wobei im Falle von 3 Rührkesseln im dritten die gleiche Temperatur wie im zweiten oder eine höhere Temperatur eingehalten wird und von der beim ersten Rührkessel verbleibenden Alkalimetallhydroxid-Restmenge dem zweiten Kessel 70 bis 95 Mol-% von dieser Restmenge und dem dritten Kessel die beim zweiten auf die stöchiometrische Alkalimetallhydroxid-Menge noch verbleibende Restmenge zugeführt wird.

## Claims

1. A continuous process for the preparation of dimethyldiallylammonium chloride by reaction, with stirring, of dimethylamine, allyl chloride and alkali metal hydroxide which is dissolved in water, which comprises carrying out the reaction in at least two stirred vessels arranged in the form of a cascade, proceeding in such a manner that

a) dimethylamine and allyl chloride in the stoichiometric amount in each case and only 60 to 95 mol% of the stoichiometrically necessary amount of alkali metal hydroxide are fed simultaneously and continuously to the first stirred vessel, and, at a temperature of 20 to 70°C and at the pressure produced, a residence time is set in this vessel such that the product leaving the vessel still contains at most 10 mol% of free amine, relative to the molar amount of dimethylamine employed,

b) the remaining amount of alkali metal hydroxide which is lacking in the first stirred vessel compared to the stoichiometric amount is fed continuously to the stirred vessels succeeding the first vessel and, at a temperature which is in the range from 20 to 70°C and which is up to 30°C higher than in the respective previous vessel and at the pressure produced, a residence time is set in each of these vessels such that the product leaving the vessel still contains at most 2 mol% of free amine in each case, relative to the molar amount of dimethylamine employed, this amount of free amine being held the same or smaller from vessel to vessel in the case of several subsequent stirred vessels, and

c) the desired dimethyldiallylammonium chloride is recovered from the product leaving the stirred vessel cascade.

2. A process as claimed in claim 1, wherein the same temperature as in the first stirred vessel or a temperature which is up to 30°C higher than in the first stirred vessel is maintained in the subsequent stirred vessels, the temperature from vessel to vessel being the same or increasing in essentially identical amounts in the case of several subsequent stirred vessels.

3. A process as claimed in one of claims 1 or 2, wherein a temperature of 30 to 50°C is maintained in the first stirred vessel and a temperature of 50 to 60°C is maintained in the stirred vessels succeeding the

first stirred vessel, the temperature from vessel to vessel being the same or increasing in essentially identical amounts in the case of several subsequent stirred vessels.

4. A process as claimed in one or more of claims 1 to 3, wherein the remaining amount of alkali metal hydroxide lacking in the first stirred vessel in comparison to the stoichiometric amount is fed to the stirred vessels succeeding the first vessel in portions which decrease from vessel to vessel.

5. A process as claimed in one or more of claims 1 to 4, wherein the reaction is carried out in two or three stirred vessels arranged in the form of a cascade.

6. A process as claimed in one or more of claims 1 to 5, wherein the reaction is carried out in two or three stirred vessels arranged in the form of a cascade, proceeding in such a manner that only 70 to 90 mol% of the stoichiometrically necessary amount of alkali metal hydroxide are fed to the first stirred vessel and, at a temperature of 30 to 50°C and at the pressure produced, a residence time is set in this vessel such that the product leaving the vessel still contains 0.05 to 10 mol% of free amine, and, at a temperature of 50 to 60°C and at the pressure produced, a residence time is set in each of the stirred vessels succeeding the first stirred vessel such that the product leaving the vessel still contains 0.05 to 2 mol% of free amine in each case, the same temperature or a higher temperature being maintained in the third vessel in comparison to the second vessel in the case of three stirred vessels and, of the remaining amount of alkali metal hydroxide lacking in the first stirred vessel, 70 to 95 mol% of this remaining amount being fed to the second vessel and the remaining amount still lacking in the second vessel related to the stoichiometric amount of alkali metal hydroxide being fed to the third vessel.

**Revendications**

1. Procédé continu pour préparer le chlorure de diméthyl-diallylammonium par réaction de la diméthylamine, du chlorure d'allyle et d'un hydroxyde de métal alcalin dissous dans de l'eau, sous agitation, procédé caractérisé en ce qu'on effectue la réaction dans au moins deux réacteurs à agitateur disposés en cascade et en ce qu'on opère de la façon suivante:

a) on introduit dans le premier réacteur à agitateur, en même temps et continuellement, la diméthylamine et le chlorure d'allyle, chacun en la quantité stœchiométrique, et seulement de 60 à 95% en moles de la quantité stœchiométrique d'hydroxyde de métal alcalin et on règle le temps de séjour dans ce réacteur, à une température de 20 à 70°C et sous la pression autogène, de telle façon que le produit quittant le réacteur contienne encore au plus 10% en moles d'amine libre par rapport à la quantité molaire de diméthylamine mise en jeu,

b) on introduit continuellement dans les réacteurs à agitateur qui font suite au premier le complément, par rapport à la quantité stœchiométrique, de la quantité d'hydroxyde de métal alcalin qui a été introduite dans le premier réacteur, et on règle le temps de séjour dans chacun de ces réacteurs, à une température comprise entre 20 et 70°C et supérieure d'au plus 30°C à celle du réacteur qui précède, et sous la pression autogène, de telle façon que le produit sortant du réacteur contienne encore à chaque fois au plus 2% en moles d'amine libre, par rapport à la quantité molaire de diméthylamine mise en jeu, cette quantité d'amine libre, dans le cas de plusieurs réacteurs à agitateur successifs, étant maintenue égale ou inférieure d'un réacteur à l'autre, et

c) du produit quittant la cascade de réacteurs on isole le chlorure de diméthyl-diallylammonium cherché.

2. Procédé selon la revendication 1, caractérisé en ce que, dans les réacteurs qui font suite au premier, on maintient la même température que dans le premier ou une température supérieure d'au plus 30°C à celle qui règne dans le premier, et, dans le cas de plusieurs réacteurs successifs, on maintient, d'un réacteur à l'autre, la même température ou une température croissant par quantités à peu près égales.

3. Procédé selon l'une des revendications 1 et 2, caractérisé en ce que, dans le premier réacteur, on maintient une température de 30 à 50°C et, dans les réacteurs qui font suite au premier, une température de 50 à 60°C, la température dans le cas de plusieurs réacteurs successifs étant maintenue constante d'un réacteur à l'autre ou croissant par quantités à peu près égales.

4. Procédé selon l'une quelconque des revendications 1 à 3, caractérisé en ce que le complément d'hydroxyde de métal alcalin, par rapport à la quantité stœchiométrique, qui reste après le premier réacteur est introduite dans les réacteurs succédant au premier en des quantités qui diminuent d'un réacteur à l'autre.

5. Procédé selon l'une quelconque des revendications 1 à 4, caractérisé en ce que la réaction est exécutée dans deux ou trois réacteurs à agitateur disposés en cascade.

6. Procédé selon l'une quelconque des revendications 1 à 5, caractérisé en ce qu'on effectue la réaction dans deux ou trois réacteurs à agitateur disposés en cascade, on n'introduit dans le premier réacteur que de 70 à 90% en moles de la quantité stœchiométrique d'hydroxyde de métal alcalin et on règle dans ce réacteur, à une température de 30 à 50°C et sous la pression autogène, un temps de séjour tel que le produit sortant du réacteur contienne encore de 0,05 à 10% en moles d'amine libre, et, dans chacun des réacteurs qui font suite au premier, on règle, à une température de 50 à 60°C et sous la pression autogène, un temps de séjour tel que le produit qui sort du réacteur contienne encore à chaque fois de 0,05 à 2% en moles d'amine libre, avec, dans le cas de trois réacteurs, les mesures supplémentaires

suivantes: on maintient dans le troisième réacteur une température égale ou supérieure à celle du deuxième, on introduit dans le deuxième réacteur de 70 à 95% en moles de la quantité d'hydroxyde de métal alcalin qui reste après le premier réacteur, et on introduit dans le troisième réacteur la quantité d'hydroxyde de métal alcalin qui reste encore après le deuxième réacteur et qui représente le complément par rapport à la quantité stœchiométrique.